# EUROPEAN PATENT SPECIFICATION

(11) **EP 0 383 759 B1**
(45) Date of publication and mention of the grant of the patent: **09.09.1998**
(21) Application number: 88904349.3
(22) Date of filing: 25.04.1988
(51) Int. Cl.: C07D 473/34, A61K 31/52

(54) **N6 SUBSTITUTED 9-METHYLADENINES: A NEW CLASS OF ADENOSINE RECEPTOR ANTAGONISTS**
N6 SUBSTITUIERTE 9-METHYLADENINE: EINE NEUE KLASSE VON ADENOSIN RECEPTOR ANTAGONISTEN
METHYLADENINES-9 SUBSTITUEES EN N6 : UNE NOUVELLE CATEGORIE D'ANTAGONISTES RECEPTEURS D'ADENOSINE

(30) Priority: 24.04.1987 US 42383
(43) Date of publication of application: 29.08.1990
(73) Proprietor: Discovery Therapeutics, Inc., Richmond, Virginia 23230-3311 (US)
(72) Inventor: OLSSON, Ray, Andrew, Tampa, FL 33606 (US)
(74) Representative: Kraus, Walter, Dr.
(86) International application number: US8801405
(87) International publication number: WO8808303

(56) References cited:
- CA-A- 0 657 337
- GB-A- 0 953 897
- GB-A- 2 041 359
- JP-B- 0 045 758
- TETRAHEDRON vol. 28, no. 3, February 1972, GREAT BRITAIN T. ITAYA ET. AL. '*Purines-6*'
- THE JOURNAL OF ORGANIC CHEMISTRY vol. 48, no. 6, 25 March 1983, pages 854 - 855 NICO J. KOS ET. AL. 'Anion Formation and Ring Opening of 9-Substituted Purines in Liquid Ammonia Containing Potassium Amide.'
- CHEMICAL ABSTRACTS, vol. 103, no. 3, 22 July 1985, Columbus, Ohio, US; abstract no. 22353q, page 554 ;column 1 ;
- CHEMICAL ABSTRACTS, Volume 107, No. 7, issued 17 August 1987, (Columbus, Ohio, U.S.A.) (D. UKENA, et al.), "N6-Substituted 9-Methyladenines: A New Class of Adenosine Receptor Antagonists", Abstract No. 51396u, FEBS Lett. 1987, 215(2), 203-8 (Eng.)
- Journal of the American Chemical Society, Vol. 79, No. 2, issued 20 January 1957, (ROBINS, et al.) "Potential Purine Antagonists. IV. Synthesis of Some 9- Methyl-6-Substituted-Purines", pages 490-494, see pages 490-494.
- Journal of the American Chemical Society, vol. 79, no. 19, issued 05 October 1957, (MONTGOMERY, et al.), "Synthesis of Potential Anticancer Agents, IX. 9- Ethyl-6-Substituted-Purines", pages 5238-5242. see all pages.
- Journal of Organic Chemistry, Vol. 28, No. 8, issued August 1963, (MYERS, et al.), "Alkylation of the Purine Nucleus by Means of Quaternary Ammonium Compounds. I. Tetraalkylammonium Hydroxidees", pages 2087-2089.
- CHEMICAL ABSTRACTS, Vol. 53, No. 15, issued 10 August 1959, (H.E. SKIPPER, et al. ), "Structure-Activity Relations and Cross-Resistance Observed on Evaluation of a Series of Purine Analogs against Experimental Neoplasms", Abstract No. 14344i-14345b, Cancer Research 19, 425-37 (1959).
- CHEMICAL ABSTRACTS, Vol. 56, No. 9, issued 30 April, 1962, (ERNST CARSTENS, et al,), "6,9-Disubstituted Purine Derivatives", Abstract No. 10167i-10168d, Ger. (East) 21,223 (Cl. 12p.) Appl. May 29, 1958.
- CHEMICAL ABSTRACTS, Vol. 68, No. 23, issued 03 June 1968, (O.N. KULAEVA, et al, ), "The Effect Produced by Variations in the Structure of Cytokinins on their Physiological Activity", Abstract No. 104032g, Dokl. Akad. Nauk, SSSR 178(5), 1204-(1968) (Russ).
- CHEMICAL ABSTRACTS, Vol. 70, No. 1, issued 06 Jan. 1969, (M.F. PETROVA, et al. ), Synthesis of Potentially Antiblastic Substances Related to 5- Hydroxytryptamine (Serotonin), Abstract No. 4056r.
- CHEMICAL ABSTRACTS, Vol. 78, No. 13, issued 02 April 1973, (KAMIYA, et al.), "4-(9H-Purin-6-yl) Amino-4-Deoxy-D-Erthyronate Esters, Abstract No. 84764y, Japan 72 45,757(Cl.C07d,A61k), 17 Nov. 1972, Appl. 70 26092, 28 Mar. 1979; 3pp.
- CHEMICAL ABSTRACTS, Vol. 79, No. 19, issued 12 Nov. 1973, (J. E. FOX et al.), "Effect of Substituents at the 9-Position on Cytokinin Activity, Abstract No. 112299s, Phytochemistry 1973, 12(7), 1531-3 (Eng.).
- CHEMICAL ABSTRACTS, Vol. 87, No. 25, issued 19 Dec. 1977, (T. HASHIZUME, et al. ), Abstract No. 195316j, "Synthesis and Biological Activity of some New 6- Benzylamino-9-Alkvlpurines," Plant Growth Subst., Proc. Int. Conf., 8th 1973 (pub. 1974) 462-7 (Eng.)
- CARDIOVASCULAR RESEARCH,1993,27,129-133
- 5th AMER. COLL. OF CARDIOL.,43rd ANNUAL SCI. SESSION,1994

## Description

### SUMMARY OF THE INVENTION

N⁶-substitution can markedly increase potency of 9-methyladenine at A₁ receptors, while having lesser effects or even decreasing potency at A₂ receptors. Effects of N⁶-substituents on adenosine receptor activity of the 9-methyladenines are reminiscent of effects of N⁶-substituents on activity of adenosine, suggesting that N⁶-substituted 9-methyladenines bind to adenosine receptors in the same orientation as do N⁶-substituted adenosines. N⁶-Cyclopentyl-9-methyladenine is more potent than 9-methyladenine.

N⁶-Cyclopentyl and several other N⁶-alkyl and N⁶-cycloalkyl analogs are selective for A₁ receptors while 9-methyladenine is the most A₂-receptor selective antagonist. The N⁶-R- and N⁶-S-(1-phenyl-2-propyl)-9-methyladenines, analogous to N⁶-R- and N⁶-S-phenylisopropyladenosine, exhibit stereoselectivity at both A₁ and A₂ receptors.

The compounds of this invention are N⁶-(endonorbornyl)-9-methyladenine and N⁶-cyclopentyl-9-methyladenine.

The preparation of 9-methyl adenines is well known. See R. K. Robins, K. J. Dille, and B. E. Christensen, J. Org. Chem., 19, 930 (1954); R. K. Robins and H. H. Lin, J. Am. Chem. Soc., 79, 490 (1957; and J. A. Montgomery and Carroll Temple, Jr., J. Am. Chem. Soc., 79, 5238 (1957).

To prepare N⁶-cyclopentyl-9-methyladenine the following additional steps were taken. A mixture of 6-chloro-9-methyladenine (0.82g), cyclopentylamine (0.52 ml), triethylamine (0.53 ml) and ethanol (60 ml), was refluxed for 24 hours. The solution was concentrated in vacuo to a yellow syrup. The syrup was passed through a C-18 column to give 0.78g or 74% yield of with m.p. - 108-109°C. ¹HNMR(Me₂SO-d6) : δ1-2(m,9 H) ; 3.7 (S,CH₃) ; 7.6(d,NH); 8.1(S,1H); 8.2(S,1H).

J. Org. Chem., vol. 28, no. 8, 1963, pp. 2087-2089 describes purine derivatives, which are substituted in the 9-position, and states that these may be regarded as structural analogs of the natural purines and are therefore of interest as potential inhibitors of biological processes.

Adenosine receptors have been divided into two subtypes, based on adenylate cyclase activity: A₁ (n₁) receptors mediate inhibition and A₂ (Rₐ) receptors mediate stimulation of adenylate cyclase activity (for reviews see ref. 1.,2). Some N⁶-substituted adenosine analogs like N⁶-R-1-phenyl-2-propyladenosine (R-PIA) have very high affinity for A₁ adenosine receptors, while 5'-N-ethylcarboxamido-adenosine (NECA) is more potent than N⁶-substituted analogs at A₂ receptors. Alkylxanthines, such as caffeine and theophylline, are the best known antagonists at adenosine receptors. Adenine was generally believed to have no effect on adenosine receptor-controlled systems. However, adenine is specific, competitive antagonist of adenosine-induced cyclic AMP accumulation in a human fibroblast cell line with A Ki of 200 µM (3). Methylation of adenine at the 9-position increases potency about 4-fold. A variety of N⁶-substituted-9-methyladenine derivatives have now been prepared and tested in three adenylate cyclase-coupled adenosine receptor systems. For A₂ adenosine receptors human platelets and rat pheochromocytoma (PC12) cells and for A₁ binding site for [³H]N⁶-R-1-phenyl-propyladenosine ([³H]PIA) was determined in rat brain membranes. Certain of the N⁶-substituted 9-methyladenines proved to be potent antagonists at adenosine receptors and some showed selectivity for either A₁ or A₂ receptors.

The invention is further illustrated by the following examples. In the following only examples 4 and 22 are within the scope of the present invention; the remaining examples are comparative examples.

The synthesis and the chemical properties of the adenine and hypoxanthine derivatives will be described elsewhere. [α-³²P]ATP (40 Ci/mmol) was purchased from Amersham (Arlington Heights, IL, USA). [³H]N⁶-R-1-Phenyl-2-propyladenosine ([³H]PIA, 49.9 Ci/mmol) was purchased from New England Nuclear, Boston, MA. USA). Other compounds used in this study were from standard sources as described (4).

Human platelet, rat pheochromocytoma (PC12) cell, rat fat cell and rat cerebral cortex membranes were prepared as described (4,5,6). Adenylate cyclase activity and binding of [³H]PIA to cerebral cortex membranes were determined essentially as described (4,5,6).

K_{B} values for the compounds were determined as described (4). Briefly stated, concentration-response curves of NECA for the stimulation of adenylate cyclase of PC12 cell and platelet membranes and of R-PIA for the inhibition of isoproterenol-stimulated adenylate cyclase activity in fat cell membranes in the absence and presence of the adenine derivative were done using at least 7 concentrations of the agonist. EC₅₀ and IC₅₀ values for the agonists were obtained from the concentration-response curves by linear regression after logit-log transformation. K_{B} values of the antagonists were calculated using the Schild equation K_{B} = C/(CR-1), where C denotes the concentration of the competitor and CR the ratio of the EC₅₀ and IC₅₀ values in the presence and absence, respectively, of the competitor. IC₅₀ of the compounds for inhibition of [³H]PIA binding to cerebral cortex membranes were transformed into Kᵢ values as described (6).

### RESULTS

### A₂ Adenosine Receptors

The effects of adenine and adenine analogs on A₂ receptor were studied in human platelets. In these cells, A₂ receptor-mediated stimulation of adenylate cyclase results in an inhibition of aggregation (5,7).

Adenine (compound Example 1) itself does not affect basal adenylate cyclase activity (data not shown), but antagonizes the NECA-induced stimulation of adenylate cyclase activity. However, adenine (Example 1) is a very weak antagonist at A₂ receptors of platelets.

Incorporation of a methyl group at the 9-position of the adenine molecule results in a marked increase in potency. Thus, 9-methyladenine (Example 2) is more potent than the adenine itself at A₂ receptors of platelets.

Substituents at the N⁶-position of 9-methyladenine (Example 2) markedly influence the antagonist potency at the platelet A₂ receptor. The N⁶-cycloalkyl analogs (Examples 3,4,6) are more potent than 9-methyladenine itself.

Incorporation of an additional methyl group into N⁶-cyclopentyl-9-methyladenine (Example 4) so as to yield a tertiary carbon at the N⁶-nitrogen reduces potency with N⁶-(l-methylcyclopentyl)-9-methyladenine (Example 5) being less potent than the parent cyclopentyl analog (Example 4). The N⁶-methyl analog (Example 7) is much less potent than 9-methyladenine at the platelet receptor, while the N⁶-3-pentyl analog (Example 8) is more potent. The N⁶-phenyl analog (Example 9) is equipotent to 9-methyladenine. The presence of ortho-fluoro moiety in compound Example 10 increases potency at the platelet A₂ receptor. The N⁶-benzyl and N⁶-(2-phenethyl analog) (Examples 11,12) are less potent than 9-methyladenine at the platelet receptors. N⁶-2-(3,4,5 trimethoxyphenyl-ethyl-9-methyladenine (Example 13) is as potent as 9-methyladenine. The heteroaryl analog N⁶-2-(3-pyridylethyl)-9-methyladenine (Example 14) is less potent than 9-methyladenine, while another heteroaryl analog N⁶-2-(3-thienylethyl) -9-methyladenine (Example 15) is somewhat more potent. The N⁶-1-phenyl-2-propyl derivatives are analogs containing a chiral carbon attached to the N⁶-nitrogen: The R-isomer (Example 16) is more potent than the S-isomer (Example 17). The O⁶-phenyl derivatives of 9-methylhypoxanthine (Examples 18-21) are very weak or inactive as inhibitors of NECA-induced stimulation of adenylate cyclase activity of human platelet membranes.

The potencies of the adenine derivatives are determined in a similar manner for A₂ receptors of rat pheochromocytoma (PC12) cells (4,8,9), using antagonism of the NECA-induced stimulation of adenylate cyclase activity of the PC12 cells membranes to assess antagonist potencies.

While there are similarities, there are also some notable differences in the structure-activity relationship for the adenines at A₂ receptors of platelets of PC12 cells.

As was the case for the platelet system, adenine (Example 1) is a very weak antagonist of the NECA-stimulated adenylate cyclase in PC12 cell membranes. 9-methyladenine (Example 2) is equally potent at A₂ receptors of human platelets and rat PC12 cells.

In contrast to the results with platelets, incorporation of N⁶-substituents into 9-methyladenine does not in any case increase the potency of the 9-methyladenine at the A₂ receptor of PC12 cells:

Potencies of all of the N⁶-substituted 9-methyladenines at A₂ receptors of PC12 cells are either the same as or lower than that of the parent compound.

In certain cases, namely the N⁶-cyclopentyl (Example 4), N⁶-3-pentyl (Example 8) and N⁶-phenyl (Example 9) analogs, the analog is more potent at the platelet A₂ receptor than at the PC12 A₂ receptor. In no case is the N⁶-substituted 9-methyladenine less potent at the platelet A₂ receptor than at the PC12 A₂ receptor. Incorporation of an additional methyl to yield the tertiary analog N⁶-1-methylcyclopentyl-9-methyladenine (Example 5) reduces potency in PC12 cells.

The O⁶-phenyl-9-methylhypoxanthines (Examples 18-21) are nearly inactive in both cell types.

### A₁ Adenosine Receptors

Rat fat cells were used for evaluation of structure-activity relationships of adenine derivatives at adenylate cyclase-coupled A₁-adenosine receptors. In these cells, adenosine analogs cause an inhibition of adenylate cyclase activity and lipolysis (10).

Adenine (Example 1) itself does not affect R-PIA-induced inhibition of fat cell adenylate cyclase activity. 9-Methyladenine (Example 2) antagonizes the effect of R-PIA.

Incorporation of cycloalkyl or alkyl substituents into the N⁶-position of 9-methyladenine (Example 2) can markedly increase the antagonistic potency at the fat cell A₁ receptor. Thus, the N⁶-cycloalkyl-9-methyladenines (Examples 3,4,6) are more potent than the parent compound 9-methyladenine and N⁶-3-pentyl-9-methyladenine (Example 8) is more potent than the parent compound at A₁ receptors of fat cells. N⁶-Methylcyclopentyl-9-methyladenine (Example 5) is less potent than the N⁶-cyclopentyl analog (Example 4). The N⁶-methyl analog (Example 7) is less potent than 9-methyladenine. The two N⁶-phenyl analogs (Example 9,10) are more potent than 9-methyladenine in the fat cell.

The N⁶-2-phenethyl (Example 12) analog is much less potent. Of the phenethyl (Examples 12,13) and heteroarylethyl (Examples 14,15) analogs only the N⁶-2-(3-thienylethyl)-9-methyladenine is more potent than 9-methyladenine itself in the fat cell. The R- and S-isomers of N⁶-1-phenyl-2-propyl-9-methyladenine (Examples 16,17) are more potent than the parent compound at A₁ receptors of fat cells. The O⁶-phenyl-9-methylhypoxanthines (Examples 18-21) are very weak or inactive as antagonists in fat cell membranes.

Similar results are obtained when the Kᵢ-values of the adenine derivatives for inhibition of [³H]PIA binding to rat cerebral cortex membranes were determined. The low potency of adenine (Example 1) is commensurate with the results from the fat cell adenylate cyclase assay. 9-Methyladenine (Example 2) is equally potent at A₁ receptors of fat cells and cerebral cortex. The Kᵢ-values of the N⁶-substituted 9-methyladenine derivatives for inhibition of radioligand binding in brain membranes are lower than the corresponding K_{B}-values from the fat cell adenylate cyclase. As in the case with fat cells, N⁶-cyclopentyl-9-methyladenine (Example 4) is more potent than the N⁶-methylcyclopentyl analog (Example 5). The 2-phenethyl (Examples 12,13) analogs are very weak antagonists of [³H]PIA binding in rat brain membranes as expected from results with fat cell adenylate cyclase. N⁶-R-1-Phenyl-2-propyl-9methyladenine (Example 16) is more potent than the S-isomer (Example 17). The O⁶-phenyl-9-methylhypoxanthinederivatives (Examples 18-21) only marginally inhibit [³H]PIA binding.

### Discussion

Xanthines, the major structural class of antagonists for adenosine receptors, have a planar heterocyclic ring system analogous to the heterocyclic purine (adenine) ring of adenosine. It has been proposed that the site in adenosine receptors that interacts with the adenine ring of adenosine also interacts with the xanthine ring of such adenosine antagonists as theophylline and caffeine (2). A variety of other compounds containing a planar heterocyclic ring have antagonistic activity at adenosine receptors. These include pyrazolopyrimidines (11), pyrazolopyridines (12,13), mesoionic xanthine analogs (14), benzopteridines (3), and 9-methyladenine (3). The last heterocycle 9-methyladenine, because of the identity of the heterocyclic ring with that of adenosine, seems even more likely than other heterocycles to bind at the same "heterocycle" site as do the adenosines. The present study was designed to test the premise that, as in the case of adenosine, N⁶-substituents on 9-methyladenine would alter activity of the 9-methyladenines in the same way as N⁶-substituents alter the activity of adenosines. The topography of binding site for N⁶-substituents in both A₁ and A₂ adenosine receptors has been extensively investigated (see 9, 16 and ref. therein). The binding site for N⁶-substituents differs significantly for A₁ receptors compared to A₂ receptors. At the A₁ receptors, N⁶-substituents can markedly enhance activity. The steroselectivity for compounds such as R-PIA and S-PIA that contain chiral N⁶-substituents is a well-known characteristic of A₁ receptors. At the A₂ receptors,most N⁶-substituents reduce activity of adenosine and steroselectivity is less pronounced than at A₁ receptors. Certain N⁶-substituents do markedly enhance activity of 9-methyladenine at A₁ receptors. The N⁶-cycloalkyl-9-methyladenines (Ex. 3,4,6) are the most potent of N⁶-substituted 9-methyladenines at A₁ receptors.

Similarly, N⁶-cycloalkyladenosines are among the most potent N⁶-substituted adenosines of A₁ receptors (9). Introduction of an additional methyl to N⁶-cyclopentyl-methyladenine (Ex. 4) to yield N⁶-1-methylcyclopentyl 9-methyladenine (Ex. 5) reduces activity at A₁ receptors. Similarly, activity of the N⁶-(1-methylcyclopentyl)-adenosine at A₁ receptors is reduced compared to N⁶-cyclopentyladenosine (9). The only N⁶-alkyl or N⁶-cycloalkyl substituent that reduces activity of 9-methyladenines at A₁ receptors is methyl, reminiscent of the low activity of N⁶-methyladenosine at A₁ receptors (9). The modest activity of N⁶-benzyl-9-methyladenine (Ex. 11) is also consonant with the low activity of N⁶-benzyladenosine at A₁ receptors (9).

The R- and S-enantiomers of N⁶-(1-phenyl-2-propyl)-9-methyladenine, analogous to R- and S-PIA, these analogs are true enantiomers, since the other chiral centers of the ribose moiety are absent. It should be noted that the stereoselectivity of the 9-methyladenine analogs at A₁ receptors is much less than the stereoselectivity of R- and S-PIA at A₁ receptors (9).

The results indicate that 9-methyladenines show effects of N⁶-substituents on activity at A₁ receptors similar to, but not identical with the effects of N⁶-substituents on agonist activity of adenosines at A₁ receptors. The near lack of activity of the four O⁶-phenyl substituted-9-methylhypoxanthines is reminiscent of the inactivity of purine ribosides containing oxygen or sulfur in place of nitrogen at the 6-position at adenosine receptors (15).

At A₂ receptors, N⁶-substituents have much smaller effects on activity of 9-methyladenine than was the case with A₁ receptors. Indeed, many substituents have no effect or reduce activity. The two A₂ receptors do not appear identical in terms of interaction with the N⁶-substituted 9-methyladenines. Whether such differences are related to species or tissue are unknown. Certainly, brain A₁ receptors differ markedly in agonist/antagonist activity in different species (16). At the A₂ receptor of human platelets the cycloalkyl-, 3-pentyl-, 2-fluorophenyl-, 2-(3-thienylethyl)-and R-1-phenyl-2-propyl-substituents enhance activity. Certain N⁶-substituted adenosines corresponding in structure to the N⁶-substituted 9-methyladenines have been investigated as agonists at platelet A₂ receptors (9). There was only a modest range of potency with the N⁶-cyclobutyl-, N⁶-cyclohexyl-, N⁶-2-(3-thienylethyl)- and N⁶-benzyl-adenosines and R-PIA being the more potent of the N⁶-substituted adenosines. Thus, the results with N⁶-substituted 9-methyladenines at platelet A₂ receptors would not have been predicted from the effects in the analogous adenosines. At the A₂ receptors of PC12 cells, none of the N⁶-substituents increased activity relative to 9-methyladenine itself. Indeed, certain substituents decreased activity. Again, these effects would not have been predicted from the agonist activity of the analogous N⁶-substituted adenosines at A₂ receptors of PC12 cells (9): As for the platelet A₂ receptor there was not a wide range of potencies for the adenosines at PC12 receptors with the N⁶-cyclobutyl-, R-PIA, N⁶-2-phenethyl-, N⁶-cyclohexyl, -N⁶-2-(3-pyridylethyl) - and N⁶-2-(3,4,5-trimethoxyphenylethyl)- analogs being the more potent of the series. Thus, the effects of N⁶-substitution on activity of 9-methyladenines and adenosines at A₂ receptors are not identical, perhaps reflecting the lack of major positive contributions of such substituents to activity at A₂ receptors. It is of interest that data on both the antagonist series of N⁶-substituted adenine and the agonist series of N⁶-substituted adenosines (9) provide evidence for the lack of identity of A₂ receptors in platelets and PC12 cells. The O⁶-phenyl-9-methylhypoxanthines are inactive or nearly so at the A₂ receptors, as is the case with 6-phenoxypurine riboside at coronary A₂ receptors (17).

Certain of the N⁶-substituted 9-methyladenines are selective for A₁ receptors, in particular N⁶-cyclo-butyl, cyclopentyl-, 1-methylcyclopentyl-, and cyclohexyl analogs, while 9-methyladenine and N⁶-2-(3,4,5-trimethoxyphenylethyl)-9-methyladenine exhibit a selectivity for A₂ receptors.

It is known that A₁ receptors influence inhibition of adenylate cyclase in fat brain and heart cells; whereas A₂ receptors influence stimulation of adenylate cyclase in endothelial and smooth muscle cells. (See John W. Daly, et al., "Structure - Activity Relationship for N⁶-Substituted Adenosines at a Brain A₁-Adenosine Receptor With A Comparison to an A₂-Adenosine Receptor Regulating Coronary Blood Flow," Biochemical Pharmacology, Vol. 35. No. 15, pp. 2467-2471 (1986)).

To prove the selectivity of these compounds, in vitro assays were conducted utilizing model tissues that are thought to contain homogenous populations of either the A₁ or A₂ adenosine receptor. The drugs were characterized by their ability to antagonize competitively the action of adenosine agonists in eliciting two responses: the reduction in force of contraction of guinea pig atrium (A₁); and the decrease in the contractile tone of the guinea pig taenia caecum (A₂).

The left atria from male guinea pigs were isolated, suspended between two punctate electrodes, and placed in a 20 ml organ bath that contained Krebs-Hensileit solution that was continuously gassed with 95% O₂ + 5% CO₂ and maintained at 31°C. The resting tension was one gram. Theatria were stimulated electrically at 1 hz, 1 ms duration pulses at supramaximal voltage. The force of contraction was recorded isometrically.

Taenia from the guinea pig caecum were cut into lengths of 1.5-2 cm. The tissues were suspended in a 20 ml organ bath containing de Jalon's solution that was gassed with 95% O₂ + 5% CO₂ and maintained at 31°C. The resting tension was 1.5 g. The contractile response was measured isotonically. Tissues were contracted with 10⁻⁷ M 5-methylfurmethide and allowed sufficient time to reach a stable contraction before addition of adenosine agonists.

The ability of the compounds to antagonize the effects of agonists was analyzed using modified Schild plots.

Although there was some sensitization of the tissue, i.e. addition of the agonist produced a larger response in the presence of high concentrations of the subject compounds, Examples 8, 4 and 22 did not competitively antagonize the effects of adenosine agonists in relaxing the taenia caecum. There was some sensitization of the tissue. This sensitization effect is also observed when using high concentrations of 8-phenyltheophylline (8-PT), a non' selective adenosine receptor antagonist. 8-PT did antagonize the effects of agonists at low concentrations. The lack of competitive antagonism suggests that the compounds tested do not interact appreciably with A₂-adenosine receptors.

However, Examples 8, 4, 22 and 23 all were found to be competitive antagonists at adenosine receptors in the atria. Examples 8 and 23 also produced increases in basal force of contraction in the atria. Affinity constants (pK_{B}) were determined for the present compounds using known methods and were as follows.

| Drug | pK_{B} |
|---|---|
| Ex. 8 | 5.4 ± 0.14 |
| Ex. 4 | 6.17 ± 0.11 |
| Ex. 22 | 6.28 ± 0.09 |
| Ex. 23 | 5.36 ± 0.1 |

These results show that the compounds of the invention display selectivity towards the A₁ adenosine receptor, with Ex. 22 being the most potent antagonist.

In vitro selectivity of the present antagonists was confirmed by in vivo tests on rate heart rate and blood pressure, the former associated with A₁ receptors and the latter associated with A₂ receptors.

Rats were anesthetized with urethan and blood pressure was monitored via a carotid cannula. Drug injections were made intravenously through a jugular cannula. Blood pressure, EGC, and heart rate were recorded on a Grass polygraph.

Adenosine produced a dose dependent decrease in blood pressure and heart rate, with a concomitant increase in the P-R interval of the ECG. Administration of N⁶-(endo norbornyl)-9-methyladenine attenuated the effects of subsequently administered adenosine on all parameters measured. At high doses, adenosine causes heart block; this effect was also substantially reduced by the agonist due to the short duration of action and route of administration of adenosine, it is often difficult to determine whether adenosine decreased blood pressure by causing peripheral vasocilation or by reducing cardiac output. Therefore NECA (5'-N-ethylcarboxamide adenosine) was used as an adenosine agonist due to its longer duration of action and A₂-adenosine receptor selectivity. Prior administration of N-0861 attenuated the effects of NECA on the heart while minimally affecting the NECA-induced decrease in blood pressure. These results show that N⁶-endo norbornyl)-9-methyladenine is a cardioselective adenosine receptor antagonist in vivo and support the date above showing selectively of the N-6 substituted 9 methyladenine as A₁ adenosine receptor antagonists.

Further investigation of this new class of adenosine receptor antagonists both in vitro and in vivo will be required to establish their usefulness in definition and elucidation of functions of adenosine receptors.

| Example No. | Compound |
|---|---|
| 1 | Adenine |
| 2 | 9-Methyladenine- (9-MA) |
| 3 | N⁶-Cyclobutyl-9-MA |
| 4 | N⁶-Cyclopentyl-9-MA |
| 5 | N⁶-Methylcyclopentyl-9-MA |
| 6 | N⁶-Cyclohexyl-9-MA |
| 7 | N⁶-Methyl-9-MA |
| 8 | N⁶-3-Pentyl-9-MA |
| 9 | N⁶-Phenyl-9-MA |
| 10 | N⁶-2-Fluorophenyl-9-MA |
| 11 | N⁶-Benzyl-9-MA |
| 12 | N⁶-2-Phenethyl-9-MA |
| 13 | N⁶-2-(3,4,5-Trimethoxyphenylethyl-9-MA |
| 14 | N⁶-2-(3-Pyridylethyl) -9-MA |
| 15 | N⁶-2-(3-Thienylethyl)-9-MA |
| 16 | N⁶-R-1-Phenyl-2-propyl-9-MA |
| 17 | N⁶-S-1-Phenyl-2-propyl-9-MA |
| 18 | O⁶-Phenyl-9-Methyhypoxanthine (9MH |
| 19 | O⁶-(2-Fluorophenyl) -9-MH |
| 20 | O⁶-(3-Fluorophenyl) -9-MH |
| 21 | O⁶-(4-Fluorophenyl) -9-MH |
| 22 | N⁶-(endo norbornyl)-9-MA |
| 23 | N⁶-4-(-2-thienyl) -3-butyl) -9-MA |
| 24 | N⁶-(exo norbornyl)-9-MA |

Only examples 4 and 22 are within the scope of the present invention. The remaining examples are comparative examples.

### References

(1) Daly, J. W. (1982) J. Med. Chem. 25, 197-207.
(2) Daly, J.W. (1985) In: Advances in Cyclic Nucleotide and Protein Phosphorylation Research (D. M. F. Cooper and K. B. Seamon, eds), Volume 19, Raven Press, New York, pp. 29-46.
(3) Bruns, R. F. (1981) Biochem. Pharmacol. 30, 325-333.
(4) Ukena, D., Daly, J. W., Kirk, K. L., and Jacobson, K. A. (1986) Life Sci. 38, 797-807.
(5) Ukena, D., Boehme, E., Schwabe, U. (1984) Naunyn-Schmiedebert's Arch. Pharmacol. 327, 36-42.
(6) Jacobson, K. A., Ukena, D., Kirk, K. L., and Daly, J. W. (1986) Proc. Natl. Acad. Sci. USA 83, 4089-4093.
(7) Cusack, N. J. and Hourani, S. M. (1981) Br. J. Pharmacol. 72, 443-447.
(8) Ukena, D., Shamin, M. T., Padgett, W., and Daly, J. W. (1986) Life Sci. 39, 743-750.
(9) Ukena, D., Olsson, R. A., and Daly, J. W. (1987) Can. J. Physiol. Pharmacol., in press.
(10) London, C. , Cooper, D. M. F., and Wolff, J. (1980) Proc. Natl. Acad. Sci. USA 77, 2551-2554.
(11) Davies, L. P., Chow, S. C., Sherrit, J. H., Brown, D. J., and Johnston, G. A. R. (1984) Life Sci. 34, 2117-2128.
(12) Williams, M., Risley, E. A., and Huff, J. R. (1981) Can. J. Physiol. Pharmacol. 59, 897-900.
(13) Psychoyos, S., Ford, C. J., and Phillios, M. A. (1982) Biochem. Pharmacol. 31, 1441-1442.
(14) Glennon, R. A., Tojani-Butt, S. M., Padgett, W., and Daly, J. W. (1984) J. Med. Chem. 27, 1364-1367.
(15) Daly, J. W., Padgett, W., Thompson, R. D., Kusachi, S., Bugni, W. J., and Olsson, R. A. (1986) Biochem. Pharmacol. 35, 2467-2481.
(16) Ukena, D., Jacobson, K. A., Padgett, W. L., Ayala, C., Shamim, M. T., Kirk, K. L., Olsson R. A., and Daly, J. W. (1986) FEBS Lett. 209, 122-128.
(17) Kuzachi, S., Thompson, R. D., Bugni, W. J., Yamada, N., and Olsson, R. A. (1985) J. Med. Chem. 28, 1636-1643,

## Claims

1. The compound N⁶-(endonorbornyl) -9-methyladenine.

2. The compound N⁶-cyclopentyl-9-methyladenine.

3. The compound of claim 1 or 2 for use in a method of antagonizing the A₁-adenosine receptor.

4. The compound of claim 1 or 2 for use in a method of reducing adenosine-induced heart block.

## Patentansprüche

1. Die Verbindung N⁶- (Endonorbornyl) -9-methyladenin.

2. Die Verbindung N⁶-Cyclopentyl-9-methyladenin.

3. Verbindung nach Anspruch 1 oder 2 zur Verwendung in einem Verfahren, bei dem dem A₁-Adenosin-Rezeptor entgegengewirkt wird.

4. Verbindung nach Anspruch 1 oder 2 zur Verwendung in einem Verfahren, bei dem der Adenosin-induzierte Herzblock vermindert wird.

## Revendications

1. Composé N⁶-(endonorbornyl)-9-méthyladénine.

2. Composé N⁶-cyclopentyl-9-méthyladénine.

3. Utilisation d'un composé selon la revendication 1 ou 2 comme antagoniste du récepteur de l'adénosine A₁.

4. Utilisation d'un composé selon la revendication 1 ou 2 pour réduire le blocage du coeur induit par l'adénosine.
